# EUROPÄISCHE PATENTANMELDUNG

(11) **EP 1 466 986 A1**
(43) Veröffentlichungstag der Anmeldung: **13.10.2004**
(21) Anmeldenummer: 04008734.8
(22) Anmeldetag: 13.04.2004
(51) Int. Cl.: C12Q 1/22

(54) **Verfahren zum Nachweis der Wirksamkeit einer Gassterilisation, insbesondere der Sterilisation mit Ethylenoxid**

(30) Priorität: 09.04.2003 DE 10316706
(71) Anmelder: Herbener, Heinz-Gerd, 52249 Eschweiler (DE); Latza, Reinhard, Dr. med., 66386 St. Ingbert (DE); Borowsky, Richard, Dipl.-Kfm., 52249 Eschweiler (DE)
(72) Erfinder: Backes, Herbert, 53424 Remagen (DE)
(74) Vertreter: Patentanwaltskanzlei Liermann-Castell

(57) **Zusammenfassung**

Bei einem Verfahren zum Nachweis der Wirksamkeit einer Gassterilisation werden Sporen oder Enzyme oder ein Medium, das Stoffe aufweist, die dem inneren Aminosäurepool gleichen und zusätzlich einen Redoxindikator besitzen Ethylenoxid ausgesetzt und anschließend einem chromogenen oder fluorogenen Nachweis unterzogen.

Vorteilhaft ist es, wenn die Enzyme oder das Medium einen Barriereschutz aufweisen.

Mit dem erfindungsgemäßen Verfahren lässt sich der Nachweis in wenigen Minuten erbringen

## Beschreibung

Die Erfindung betrifft ein Verfahren zum Nachweis der Wirksamkeit einer Gassterilisation, insbesondere der Sterilisation mit Ethylenoxid.

Für die Sterilisation von Medizinprodukten gilt die europäische Norm EN 550, die insbesondere die Validierung und Routineüberwachung für die Sterilisation mit Ethylenoxid betrifft. In der Einleitung dieser Norm wird die gesamte Problematik einer Sterilisation von Medizinprodukten dargestellt und es werden begleitende Normen erwähnt. Da sich die Sterilität - wie in der Norm erwähnt - von Produkten nur dann nachweisen lässt, wenn man jenes einzelne Produkt auf Sterilität testet, sind alternative Methoden zugelassen. Ohne Anspruch auf Vollständigkeit zu erheben werden im Folgenden zwei Verfahren kurz erläutert:
Die parametrische Freigabe (siehe EN 550)

Die parametrische Freigabe ist sehr kostenaufwändig und wird daher nur von wenigen Institutionen angewandt.
Die Validierung des Sterilisationsvorganges an sich

Dieses Verfahren beruht auf einer statistisch relevanten Verteilung von sowohl Indikatoren, die nachweisen, dass das zu sterilisierende Gut Ethylenoxid ausgesetzt war (Gasindikatoren) als auch auf Indikatoren, die nachweisen, dass der Sterilisationsvorgang wirkungsvoll gewesen ist. Das Verfahren ist daher sehr zeit- und arbeitsaufwendig. Der Nachweis, dass das Sterilisationsgut Ethylenoxid ausgesetzt war, beruht auf einer Farbreaktion. Diese Reaktion hat zwei Nachteile. Sie ist weder ausreichend empfindlich, noch gibt sie auch nur annähernd einen Anhaltspunkt dafür, wie lange das Gas auf das Sterilisationsgut eingewirkt hat. Der Grund hierfür liegt darin, dass die Reagenzien zum Nachweis des Ethylenoxids normalerweise auf Papierstreifen aufgebracht sind. Aufgrund der Gesetze der Diffusion erlaubt diese Methode keine Aussage darüber, wie lange das Sterilisationsgut dem Ethylenoxid ausgesetzt war.

Zum Nachweis der Sterilisation dienen Papierstreifen mit Sporen von Bacillus subtilis oder alternative Träger, die mit Sporen dieser oder einer anderen Art beschichtet sind. Laut der Definition der Sterilität darf keine dieser Sporen auskeimen. Der Zeitraum um sicherzugehen, dass alle Sporen abgetötet wurden, erfordert eine Inkubation der Sporensuspension oder des Trägers mit den Sporen über einen Zeitraum von 2 bis 7 Tagen in einem geeigneten Medium.

Der Erfindung liegt die Aufgabe zu Grunde, ein Verfahren zum Nachweis der Wirksamkeit einer Gassterilisation, insbesondere der Sterilisation mit Ethylenoxid, bereitzustellen, dass schneller als die bekannten Verfahren den Nachweis ermöglicht.

Bei dem erfindungsgemäßen Verfahren werden Sporen oder Enzyme oder ein Medium, das Stoffe aufweist, die dem inneren Aminosäurepool gleichen und zusätzlich einen Redoxindikator besitzen, dem Gas ausgesetzt und anschließend einem chromogenen oder fluorogenen Nachweis unterzogen. Hierbei werden vorzugsweise hitzestabile Enzyme verwendet. Dies erlaubt es, beim Sterilisieren neben Gas auch Hitze zu verwenden. Eine Steigerung des Sterilisationseffektes ist schon bei Temperaturen über 40°C, vorzugsweise über 60 oder 80°C und besonders bevorzugt über 100° C zu erreichen. Die Enzyme sollten hierbei nur einen Aktivitätsverlust von maximal 5 % erleiden. Beispiele sind 4 h bei 85 °C (Amylase), 9 h bei 55 °C oder 5 h bei 95 °C (Protease). Es sind vor allem auch in Waschmitteln verwendete Enzymarten von Interesse, da auch hier eine gute Aktivität auch noch bei höheren Temperaturen verlangt wird.

In dem Prozess der Keimung sind Enzyme involviert, die Proteine oder Heteropolysacharide hydrolisieren. Die Wirkung dieser Enzyme lässt sich durch chromogene oder fluorogene Substrate nachweisen. Durch den Einsatz solcher Substrate, zum Beispiel eines Substrats für ein Trypsin-like-Enzyme oder eines Substrates, das eine N-acetylhexosaminidase nachweist, wird die Inkubationszeit stark verkürzt.

Weiterhin wird vorgeschlagen, dass das Medium Stoffe enthält, die dem inneren Aminosäurepool gleichen und zusätzlich einen Redoxindikator (chromogener oder fluorogener Natur) besitzen, der frühzeitig ein Wachstum oder eine Keimung anzeigt.

Die biologische Wirkung von Ethylenoxid beruht vor allem in der Inaktivierung von Enzymen. Dies geschieht zum Beispiel durch eine Reaktion mit funktionellen Gruppen wie NH₂, SH, OH, COOH oder C=O. Durch diese Reaktion werden auch Sekundär-, Tertiär- oder Quartärstrukturen von Enzymen zerstört.

Die biologische Wirksamkeit von Ethylenoxid lässt sich auch wie folgt nachweisen: man kann ein Enzym oder Enzymsubstrat auf irgendeinen Träger aufbringen und der Wirkung des Ethylenoxids aussetzen. Durch die biologische Wirksamkeit des Ethylenoxids werden sowohl Enzym als auch Enzymsubstrat zerstört. Folglich können, gleich welche Kombination man anwendet, Enzym und Enzymsubstrat weder zu chromogenen noch fluorogenen Nachweisen ihrer Funktion herangezogen werden.

Vorteilhaft ist es, wenn durch den Einsatz von Aminosäuren überlebende Sporen spontan zur Keimung getriggert werden. Dies lässt sich durch den Einsatz bestimmter Aminosäuren erreichen.

Da das aktive Gas (Ethylenoxid) nur wenige Nanometer in die Cortex (,,die Wand") einer Bacillusspore eindringt, ist es auch denkbar, das Enzym oder das Enzymsubstrat in der gleichen Weise zu schützen, wie dies der Funktion der Cortex entspricht. Es wird daher vorgeschlagen, dass die Enzyme oder das Medium einen Barriereschutz aufweisen.

Nur beispielsweise könnte man auf einem Papierstreifen β-Galaktosidase auftragen. Dieses Enzym spaltet Lactose in Glycose und Galaktose. Das Enzym kann zum Beispiel durch geeignete Substanzen stabilisiert werden. Da die Wirkung des Ethylenoxids eine chemische ist, bedarf es in erster Linie eines Barriereschutzes für das Enzym oder das Substrat. Dieser Schutz kann zum Beispiel durch Algenat oder einen ähnlichen Stoff erfolgen. Es wird daher vorgeschlagen, dass der Barriereschutz ein Algenat ist.

Weiterhin wird vorgeschlagen, dass ein chromogenes oder fluorogenes Enzymsubstrat vor oder nach der Gasaussetzung zugegeben wird.

Gibt man nach der Einwirkung von Ethylenoxid auf den Träger ein chromogenes Substrat wie zum Beispiel ONPG oder PNPG oder vergleichbare chromogene oder fluorogene Substrate, kommt es zur einer Reaktion falls das Enzym noch Restwirksamkeit hat. Genauso kann man umgekehrt vorgehen und ein Enzymsubstrat gleich welcher Art auf einen Träger aufbringen und der Wirkung des Ethylenoxids aussetzen. Wird das Substrat chemisch verändert, kann es von dem Enzym, in diesem Falle β-Galaktosidase, nicht mehr gespalten werden. Die beiden genannten biochemischen Verfahren bedeuten eine Herabsetzung der Zeitgrenze für die Wirksamkeit des Ethylenoxids von zwei Tagen auf wenige Minuten.

Beim Nachweis der Wirksamkeit einer Gassterilisation besteht darüber hinaus das Problem, nachzuweisen, wie lange ein Nachweispräparat dem Gas ausgesetzt worden ist. Die im Stand der Technik verwendeten Papierstreifen reagieren mit dem Gas, sofern sie mit dem Gas in Berührung kommen. Wenn sie jedoch aus irgend welchen Gründen nicht oder nur ungenügend mit dem Gas in Berührung kommen, wurde das Nachweisverfahren nicht ordnungsgemäß durchgeführt.

Dieses Problem wird mit einem Verfahren zum Nachweis der Gassterilisation, insbesondere der Sterilisation mit Ethylenoxid gelöst, bei dem ein Reagenz an mindestens einer Seite an einer durchsichtigen gasundurchlässigen Fläche anliegt.

Vorteilhaft ist es, wenn die gasundurchlässige, durchsichtige Fläche eine Glasfläche ist. Das Gas dringt somit in das Reagenz an allen Seiten ein und nur dort nicht, wo das Reagenz an der durchsichtigen gasundurchlässigen Fläche anliegt. Sofern im Reagenz eine Verfärbung aufgrund des Gases entsteht, führt dies zu einer Verfärbung von einer Fläche des leichten Gaszutritts zu einer zentralen Fläche mit schlechterem Gaszutritt. Die Länge des verfärbten Bereichs ist somit ein Indiz für die Dauer, über die das Reagenz dem Gas ausgesetzt wurde.

Beispielsweise kann ein Papierstreifen, der mit dem Reagenz getränkt wurde, zwischen zwei Glasflächen gehalten werden. Das Gas wird somit zwischen den Glasflächen zum Papierstreifen gelangen und im Papierstreifen je nach Einwirkzeit eine kleinere oder eine größere ringförmige Verfärbung bewirken.

Vorteilhaft ist es, wenn die durchsichtige Fläche an einem porösen Körper anliegt. Der poröse Körper erlaubt es dann mit einem Reagenz getränkt zu werden und einen Gaszutritt zu erlauben, der jedoch an der Seite unterbunden ist, an der der poröse Körper an der durchsichtigen Fläche anliegt.

Eine besonders bevorzugte Ausführungsform sieht vor, dass die durchsichtige Fläche rohrförmig ist. Dies erlaubt es innerhalb des Rohres das Reagenz gegebenenfalls in einem porösen Körper anzubringen. Das Gas tritt dann entweder über eine offene Endseite des Rohres oder über zwei offene Seiten des Rohres in das Rohr ein und verfärbt je nach Einwirkdauer einen größeren oder einen kleineren Bereich des im Rohr angebrachten Reagenzes.

In einem Ausführungsbeispiel befindet sich in einem Glasrohr ein Körper oder Stoff, der gegen Ethylenoxid inert ist, aber mit einem Reagenz getränkt ist, das unter Farbgebung oder Farbänderung mit Ethylenoxid reagiert. Am Rohr lässt sich dann optisch die Diffusion des Ethylenoxids durch die Länge der Verfärbung nachweisen, aus der auf die Dauer und somit die Einwirkzeit geschlossen werden kann.

Es ist auch denkbar, dass sich Scheiben eines solchen Materials aufbringen lassen, um einer Verlängerung der Ethylenoxidwirkung gerecht zu werden.

## Patentansprüche

1. Verfahren zum Nachweis der Wirksamkeit einer Gassterilisation, insbesondere der Sterilisation mit Ethylenoxid, bei dem Sporen oder Enzyme oder ein Medium, das Stoffe aufweist, die dem inneren Aminosäurepool gleichen und zusätzlich eine Redoxreaktion besitzen, dem Gas ausgesetzt werden und anschließend emem chromogenen oder fluorogenen Nachweis unterzogen werden.

2. Verfahren nach Anspruch 1, ***dadurch gekennzeichnet, dass*** durch Einsatz von Aminosäuren überlebende Sporen spontan zur Keimung getriggert werden.

3. Verfahren nach einem der vorhergehenden Ansprüche, ***dadurch gekennzeichnet, dass*** die Enzyme oder das Medium einen Barriereschutz aufweisen.

4. Verfahren nach Anspruch 3, ***dadurch gekennzeichnet, dass*** der Barriereschutz ein Algenat ist.

5. Verfahren nach einem der vorhergehenden Ansprüche, ***dadurch gekennzeichnet, dass*** ein chromogenes oder fluorogenes Enzymsubstrat vor der Gasaussetzung zugegeben wird.

6. Verfahren nach einem der vorhergehenden Ansprüche, ***dadurch gekennzeichnet, dass*** ein chromogenes oder fluorogenes Enzymsubstrat nach der Gasaussetzung zugegeben wird.

7. Verfahren zum Nachweis der Wirksamkeit einer Gassterilisation, insbesondere der Sterilisation mit Ethylenoxid, bei dem ein Reagenz an mindestens einer Seite an einer durchsichtigen gasundurchlässigen Fläche anliegt.

8. Verfahren nach Anspruch 7, ***dadurch gekennzeichnet, dass*** die durchsichtige Fläche eine Glasfläche ist.

9. Verfahren nach Anspruch 7 oder 8, ***dadurch gekennzeichnet, dass*** die durchsichtige Fläche an einem porösen Körper anliegt.

10. Verfahren nach einem der Ansprüche 7 bis 9, ***dadurch gekennzeichnet, dass*** die durchsichtige Fläche rohrförmig ist.
